# EUROPEAN PATENT APPLICATION

(11) **EP 4 260 962 A1**
(43) Date of publication of application: **18.10.2023**
(21) Application number: 22729010.3
(22) Date of filing: 27.01.2022
(51) Int. Cl.: B21F 1/00, A61M 25/09

(54) **ANGLE SHAPING DEVICE**

(30) Priority: 11.12.2020 CN 202011448162
(71) Applicant: Zhang, Jian, Beijing 100000 (CN); Wu, Wei, Shenzhen, Guangdong 518000 (CN)
(72) Inventor: LI, Xuedong, Beijing 100000 (CN); ZHANG, Jian, Beijing 100000 (CN); WU, Wei, Shenzhen, Guangdong 518000 (CN)
(74) Representative: Tiburzi, Andrea
(86) International application number: PCT/CN2022/074449
(87) International publication number: WO 2022/122053

(57) **Abstract**

The present invention relates to a device for shaping an angle, including a support platform (2) and an angled cutter head (31). The support platform is configured to serve as a support surface for placing a guide wire. The angled cutter head is configured to press and bend the guide wire at an accurate angle. The present invention also relates to a method for shaping with a device for shaping an angle, including the following steps: placing a guide wire on a support platform, extending out the guide wire by a certain bending length, pressing down on the guide wire with an angled cutter head of which the bottom surface is provided with a certain angle, and preparing the guide wire of which one end has an angle of required accuracy. The present invention relates to a device for shaping a guide wire. A guide wire entry platform (4) is provided on a support platform. The guide wire entry platform is provided thereon with a guide wire entry hole (37) so that a guide wire is guided to be below an angled cutter head. The device for shaping the angle is capable of rapidly and reliably providing a guide wire having a certain bending angle according to requirements.

## Description

### TECHNICAL FIELD

The present invention relates to the technical field of instruments, and in particular, to a device for providing angular shaping in the technical field of a medical device.

### TECHNICAL BACKGROUND

In practice, filamentary materials of a plurality of metal materials or non-metallic materials often require to be bent at an angle to form a shape with a certain length of a bending head for ease of use. However, most of tools for angle bending in the prior art are used for a metal with a certain degree of thickness. The filamentary material is usually bent at an angle manually, which leads to a deviation in a bending angle and is not accurate. For example, in medical clinical practice, a "guide wire"-shaped material is often used in a surgery or an interventional therapy to evacuate or fix certain tissues. These guide wires have usually a diameter of less than 1 mm and have to be bent at an accurate angle at one end to achieve their function. Due to the lack of a special bending apparatus or tool, most of the guide wires are bent manually by a doctor based on visual inspection and experience. The guide wire is bent and formed generally roughly, which can easily cause an accident when precision production is required or a medical surgery and other situations require precision and accuracy. For example, regarding cardiovasology, when a chronic total occlusion (CTO) is evacuated, the head of the guide wire for evacuating a vessel requires to be bent at a certain length and angle according to the shape of the vessel, so that it is more convenient for the doctor to complete the surgery smoothly as required. However, at present, a clinician generally shapes the guide wire based on his experience, and is unable to accurately grasp a shaping length and angle, which can easily lead to failure of vascular evacuation during the surgery, causing a certain impact on the quality of the surgery and seriously threatening the health and life of a patient.

### SUMMARY

To solve the forgoing problems, the present invention provides a device for shaping an angle, which can quickly and reliably provide a guide wire with a certain bending angle as required.

The guide wire described in the present invention includes: a metal wire, a metal wire containing a coating, and a hollow metal wire, where the metal includes alloy; a non-metallic wire, a non-metallic wire containing a coating, and a hollow non-metallic wire, where the non-metal is a hard non-metallic material with certain ductility capable of performing certain penetration functions; and a guiding wire (a guiding line) which is a tool configured as puncture, insertion of a catheter, and evacuation. The guide wire acts as a guide and support for the catheter and helps the catheter enter a lumen or a target site.

To further describe the technical solution of the present invention clearly, the entry direction of the guide wire is set as from front to back. A front side and a back side mentioned in a text are understood as follows: a side close to the wire guide guided before the guide wire is entered is the front side, and a side close to the bending head of the guide wire during a bending process is the back side.

To realize the above objectives:
The present invention first provides a device for shaping an angle, including a support platform and an angled cutter head. The support platform serves as a support surface for placing the guide wire. The angled cutter head is configured to bend the guide wire at an accurate angle. The guide wire is placed on the support platform, extending a certain bending length. The guide wire is pressed down with an angled cutter head of which the bottom surface is provided with a certain angle. The guide wire of which one end has an angle of required accuracy is prepared.

The angled cutter head of the present invention is a three-dimensional structure (a cutter head body) with a specific angled bottom surface. Further, the angled bottom surface can consist of one or more different inclined surfaces (small cutter heads). Each of the inclined surfaces (the small cutter heads) has different inclined angles. Therefore, the wire guide heads with different bending angles can be bent during a wire pressing process. Preferably, the inclined surfaces are distributed continuously or at intervals.

Further preferably, the small cutter head described in the present invention can have the following structures: one or more bosses are provided on the bottom surface of the main body of the cutter head along the side thereof. the vertical side of the boss opposite to the side of the support platform is prepared as the inclined surface. The inclined surface is at a certain angle to the side of the support platform.

Further, the angled cutter head is fixedly connected to the support platform via a connecting device. The angled cutter head can move up and down to bend the guide wire under the constraint of the connecting device. The connecting device is preferably a spring connecting device. Further preferably, the spring connecting device is structured in such a way that the two ends of the angled cutter head is connected to the support platform via a first spring. Further preferred, the two ends of the angled cutter head are provided with a hollow cavity. A first spring is provided in the hollow cavity. The bottom of the first spring is fixedly connected to the support platform. To be able to stably limit the displacement of the angled cutter head other than the displacement in a vertical direction, the first spring is sleeved on a guide post. One end of the guide post is fixed to the support platform, and the other end of the guide post passes through the angled cutter head and is a free end.

Further, the device for shaping the angle of the present invention is provided with a wire guide entry platform on the support platform. The wire guide entry platform is provided thereon with a wire guide entry hole for guiding the wire guide to be below the angled cutter head. Specifically, to ensure that the guide wire can be accurately below the head of the angled cutter head after passing from one side of the support platform to the other side thereof. The guide wire entry hole is provided on one side of the support platform. Preferably, one or the plurality of guide wire entry holes can be arranged. The number of the guide guide entry holes matches that of the small cutter heads of the angled cutter head. The size of the aperture of the guide wire entry hole is matched with the thickness of a required bending material according to actual needs. The lowest point of a wire exit at the end of the guide wire entry hole is tangential with the side of the support platform to ensure that the upper side of the support platform can play a support role after the guide wire enters the guide wire entry hole.

Further, the device for shaping the angle of the present invention is provided with a wire pressing device. Specifically, the wire pressing device is provided on the angled cutter head in order not to displace the guide wire during the bending process.

Preferably, the wire pressing device is the bottom side of the angled cutter head. During the bending process of the guide wire, the angled cutter head goes down. The bottom side of the angled cutter head cooperates with the upper side of the support platform to fix the guide wire. The small cutter head located on the lower side of the main body of the angled cutter head is adjacent to the rear side of the support platform and is on the lower side of the rear side of the main body of the angled cutter head. An inclined angle is formed between the inclined surface of the small cutter head and the rear side of the support platform when the angled cutter head is pressed down.

Preferably, the angled cutter head is connected to the wire pressing device via the second spring device. Further, the main body of the angled cutter head is provided with an inner cavity. A pair of second springs are provided in the inner cavity. One end of the second spring is fixed with the cutter head body, and the other end of the second spring is fixed with the wire pressing device. The lower side of the wire pressing device is lower than the lower side of the cutter head body in the natural state of the second spring. Further, the inner cavity is provided in the middle of the angled cutter head. The second spring is provided symmetrically along the midline of the inner cavity. Further, the wire pressing device is a three-dimensional structure of which the bottom surface is a plane.

Further, the device for shaping the angle of the present invention is provided with a limit device for precisely defining the bending length of the guide wire. Specifically, a limit device is provided on the rear side of the support platform. The limit device is provided on the rear side of the angled cutter head and the opposite side of the guide wire entry hole. A distance between the front side (a limit surface) of the limit device and the rear side of the support platform is called as a traveling length. The traveling length determines the bending length of the guide wire. According to the actual needs, the structure of the limit device is adjusted or different limit devices are arranged so that different traveling lengths can be set at the same time to control the head bending length of different guide wires.

Further, the device for shaping the angle of the present invention can further be provided with a substrate. The substrate is configured to carry the device for shaping the angle.

Preferably, the device for shaping the angle is removably connected to the substrate. The connection mode includes but is not limited to: a bolt, a screw connection, snap-fitting, etc. The substrate is provided with a mounting structure of the device for shaping the angle. The mounting structure includes but is not limited to: a screw hole, a snap-fitting structure, etc. Further preferably, one or more devices for shaping the angles are simultaneously provided on the substrate. The device for shaping the angle can have the same or different shaping angles and shaping lengths.

Further preferably, the limit device is arranged on the substrate. The substrate is arranged on the rear side of the angled cutter head and the opposite side of the guide wire entry hole. There is a traveling length between the substrate and the rear side of the support platform.

Further, the device for shaping the angle can be easily combined with the substrate in use. The limit device can be integrally provided with the support platform.

Further preferably, to facilitate the preparation of the guide wires with different bending angles and lengths at any time, mounting structures of a plurality of monomers of the device for shaping the angle can be provided on the substrate. each of the devices for shaping the angles and the limit device form one monomer of the device for shaping the angle. The monomer of each of the devices for shaping the angles can has different traveling lengths, i.e., to cope with the demand for different bending angles under a single traveling length. Therefore, the device for shaping the angle formed by the plurality of monomers of the device for shaping the angle can cope with the task of bending the guide wires with requirements of different traveling lengths and different bending angles.

The present invention provides the device for shaping the angle. The device for shaping the angle can be configured to bend various types of wire guide ends. A user can adjust the traveling length and the specifications of the angled cutter head according to actual needs. According to the properties and thickness of a material to be bent, the size and the manufacturing material of the device for shaping the angle are adjusted to apply the bending material. A preferred material for the preparation of the device for shaping the angle of the present invention includes a synthetic material, an alloy material or a metallic material and so on.

Second, the present invention provides a method for shaping a device for shaping an angle, including the following steps: setting a guide wire entry hole at one side (a front side) of the support platform; setting a plurality of guide wire entry holes; cooperating, by the size of the aperture of the guide wire entry hole, with the thickness of the material to be bent according to actual needs, and the lowest point of the wire exit at the end of the guide wire entry hole being tangential with the upper side of the support platform to ensure that the upper side of the support platform can play a support role after the guide wire enters the guide wire entry hole.

The support platform is connected to the angled cutter head via a connecting device together. Specifically, the support platform can be connected to the angled cutter head together via a spring connecting device so that the angled cutter head can move up and down relative to the support platform.

Preferably, the angled cutter head includes a cutter head body and an inclined surface cutter head. The vertical side of the inclined surface cutter head adjacent to the support platform is provided as a slope surface as the cutter head. The slope surface cooperates with the upper side of the support platform during the sinking process of the angled cutter head to complete the task of bending the guide wire.

In addition, one limit device that cooperates with the other side (the rear side) of the substrate or the support platform needs to be prepared. The limit device is fixed to the rear side of the substrate or the support platform. A distance between the limit device and the rear side of the support platform is the traveling length, which is the bending length.

Preferably, the traveling length includes any desired length such as: 0.5 mm, 1 mm, 1.2 mm, 1.5 mm, 2 mm, 3 mm, or 5 mm.

Further preferably, the cutter head angle of the angled cutter head can be any desired angle, such as 15 degrees, 20 degrees, 25 degrees, 30 degrees, 40 degrees, 45 degrees, 50 degrees, 70 degrees, or 90 degrees.

Preferably, the limit device can be moved back and forth. The limit device can be withdrawn (or extruded outward) when the angled cutter head is pressed down to form, ensuring that the end of the guide wire is not blocked when the guide wire is bent at an angle.

In summary, the technical solutions used in the present invention can easily and quickly bend metal guide wires or other materials with a certain degree of toughness and ductility. Under the conception of the present invention, the structure of the device can be both small and light, as well as large. Pressure can be provided manually or via a mechanical force. The device for shaping the angle provided by the present invention can accurately bend the guide wire at an angle and a length, and can prepare guide wires with different bending lengths and bending angles according to actual needs.

### BRIEF DESCRIPTION OF THE DRAWINGS

Fig. 1 is a schematic diagram of a specific structure of the present invention.
Fig. 2 is a schematic diagram of another specific structure of the present invention.

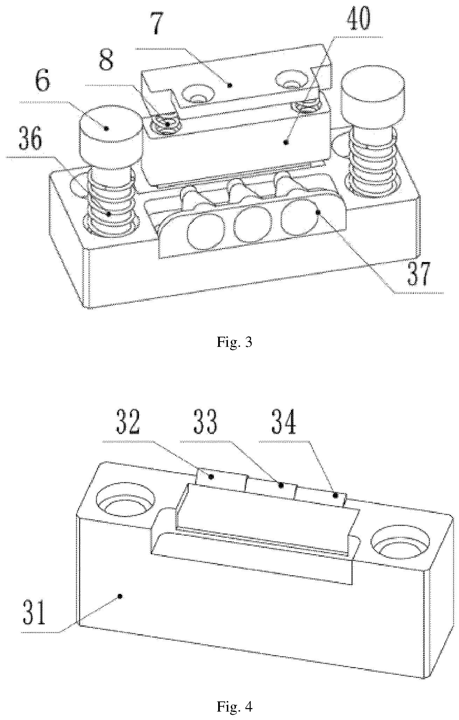

Fig. 3 is a schematic diagram of a specific internal structure of the present invention.
Fig. 4 is a schematic structural diagram of a specific angled cutter head of the present invention.

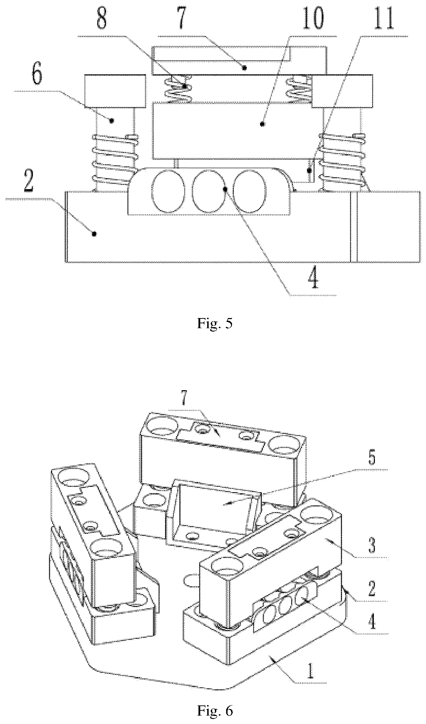

Fig. 5 is a front view of a schematic diagram of a specific internal structure of the present invention.
Fig. 6 is a schematic diagram of another specific structure of the present invention.

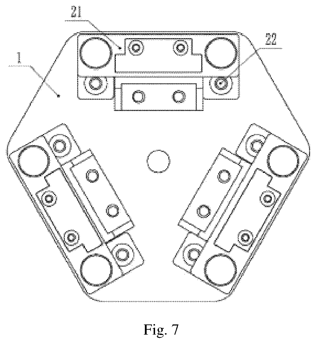

Fig. 7 is a top view of schematic diagram of a specific structure of the present invention.

### DETAILED DESCRIPTION OF EMBODIMENTS

In the following, taking as an example a guide wire commonly used in cardiovascular surgery, the technical solution of the present invention is further explained in conjunction with the drawings.

### Embodiment 1

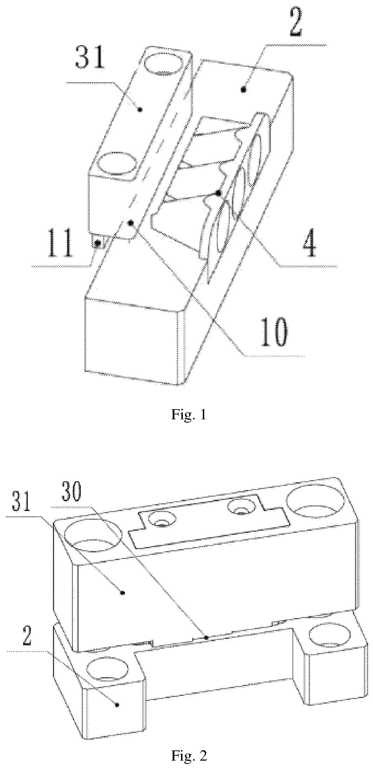

As shown in Fig. 1, a device for shaping an angle includes a support platform 2 and an angled cutter head 31. The support platform 2 is used as the lower support surface of a guide wire. The angled cutter head 31 is configured to bend the guide wire. The guide wire is placed on the support platform 2, extends a certain bending length, and pressed down against the side of the support platform with the angled cutter head 31 with a certain angle on the bottom side thereof.

The angled cutter head 31 includes a first stepped platform 10 and a second stepped platform 11. The second stepped platform 11 and the vertical side adjacent to the support platform 2 are provided as a certain angle of a slope surface as the cutter head. During the sinking of the angled cutter head 31, the bottom surface 10 of the first stepped platform fits the upper side of the support platform 2 to fix the guide wire. The upper cutter head part of the second stepped platform 11 is pressed down to complete the task of bending the guide wire. Embodiment 2

As shown in Fig. 2, a device for shaping an angle includes a support platform 2 and an angled cutter head 31. The support platform 2 is used as the lower support surface of a guide wire. The angled cutter head 31 is configured to bend the guide wire. The guide wire is placed on the support platform 2, extends a certain bending length, and pressed down against the side of the support platform with the angled cutter head 31 with a certain angle on the bottom side thereof.

The angled cutter head 31 is in the form of a long block. The rear side of the bottom of the angled cutter head is provided with a certain length of protruding points as the cutter head part 30. The vertical side of the protruding points near the rear side of the support platform is an inclined surface. These inclined surfaces fit the upper and rear sides of the support platform 2 after the angled cutter head 31 is sunk, to complete the task of bending the guiding wire.

### Embodiment 3

An device for shaping an angle includes g a support platform 2, and an angled cutter head 31 provided on the support platform 2. The angled cutter head 31 is fixed movably up and down on the upper side of the support platform 2 via the spring connecting device. The cutter head portion 30 of the angled cutter head 31 cooperates with the support platform 2 after the angled cutter head 31 is sunk, to complete a bending action.

### Embodiment 4

An device for shaping an angle includes a substrate 1 and the device 21 for shaping the angle provided on the substrate 1. The device for shaping the angle 21 includes a support platform 2 and an angled cutter head 31 provided on the support platform 2. The angled cutter head 31 is fixed movably up and down on the upper side of the support platform 2 via the spring connecting device. The cutter head portion of the angled cutter head 31 cooperates with the support platform 2 after the angled cutter head 31 is sunk, to complete a bending action. The device for shaping the angle 21 and the substrate 1 are removably connected to each other via screw connection. Three devices for shaping the angles can be provided on one substrate 1. The substrate 1 is provided thereon with a screw hole 22 of a mounting structure of the device for shaping the angle.

### Embodiment 5

An device for shaping an angle consists of a support platform 2, an angled cutter head 31, a wire guide entry platform 4, a limit device 5, and a wire pressing device 40. The angled cutter head 31 is provided with three angled bending heads of 30°, 45° and 70°, which are molded in a single die-casting via a high precision mold, to ensure that the bending angle is accurate and consistent. The two sides of the angle head 31 is also loaded with two guide posts 6 to ensure that the angle head 31 is moved accurately up and down. The guide wire entry platform 4 is provided thereon with a guide wire entry hole. 37. The angled cutter head 31 is loaded with a wire pressing device 40. When pressed down, the wire pressing device 40 first presses the guide wire on the support platform 2 to prevent the guide wire from moving. When the angled cutter head 31 is pressed to the bottom, the angled cutter head 31 cooperates with the support platform 2 to bend the guide wire to a standard angle. A wire guide limit hole is provided on the limit device 5 corresponding to the position of the wire guide entry hole 37 to prevent the wire guide end from running off or bending. Preferably, the limit device can be moved back and forth. The limit device 5 can be 5 withdrawn (or extruded) when the angled cutter head 31 is pressed down to form, ensuring that the end of the guide wire is not blocked when the guide wire is bent at an angle.

### Embodiment 6

A device for shaping an angle includes a substrate 1, a support platform 2 provided on the substrate 1, and an angled cutter head provided on the support platform 2. The angled cutter head is fixed movably up and down on the upper side of the support platform 2 via a spring connection device. The support platform 2 and three angled cutter heads with which the support platform cooperates are provided, and fixed in a Chinese character "pin" shape on the substrate 1. The rear side of the support platform 2 is provided with a limit device 5. There is a traveling length between the limit device 5 and the support platform 1. The traveling length is 1mm, 2mm, and 3mm, respectively. The angled cutter head is provided thereon with three different angled surface cutter heads 32, 33, 34 to facilitate the bending of the guide wire into a required angle. Correspondingly, the side of the support platform 2 is provided with a guide wire entry platform 4. A plurality of guide wire entry holes 37 are provided on the guide wire entry platform. If the guide wire enters from different guide wire entry hole 37, the guide wire corresponds to different angles of the cutter head.

The two sides of the angled cutter head 31 are provided with cylindrical cavities. A first spring 36 is provided inside the cylindrical cavity. Further, to be able to stably limit the displacement of the angled cutter head 31 other than the displacement in a vertical direction, a guide post 6 is provided at the inside of the first spring 36. One end of the guide post 6 is fixed to the support platform 2, and the other end of the guide post passes through the angled cutter head 31 and is a free end. The cylindrical cavity is through-hole shaped and provided with a circular boss protruding inward at the inside thereof to restrain the first spring 36 so that the first spring 36 is fixed between the angled cutter head and the support platform. The guide post passes through the circular boss.

To ensure that the wire guide can be located accurately under a cutter head part 30 of the angled cutter head 31 after passing from one side of the support platform 2 to the other side thereof, the side of the support platform is provided with the wire guide entry hole 37. There are the plurality of the wire guide entry holes 37. The number of the wire guide entry holes matches the number of cutter heads of the the angled cutter head 31. The size of the aperture of the wire guide entry hole 37 cooperates with the thickness of the material required to be bent according to actual needs. The lowest point of the end of a wire exit of the wire guide entry hole 37 is tangential with the upper side of the support platform 2, to ensure that the upper side of the support platform 2 can play a support role after the wire guide enters the wire guide entry hole 37.

In order not to displace the guide wire during a bending process, the angled cutter head is provided thereon with a wire pressing device. The wire pressing device is the bottom side of the angled cutter head. During the bending process of the guide wire, the angled cutter head 31 goes down. The bottom side of the angled cutter head cooperates with the upper side of the support platform 2 to fix the guide wire. The small cutter head part 30 located on the lower side of the main body of the angled cutter head 31 is located to the rear side of the support platform and is on the lower side of the rear side of the main body of the angled cutter head 31. An inclined angle is formed between the inclined surface of the cutter head part 30 and the rear side of the support platform when the angled cutter head 31 is pressed down.

In addition, the wire pressing device can be arranged in another way. An inner cavity of a main body of the cutter head is provided with a pair of second springs 8 symmetrically along the midline thereof. One end of the second spring 8 is fixed with the main body of the cutter head. The second spring 8 is fixed with a pressing plate 7 in this specific embodiment. The pressing plate is fixed with the main body of the cutter head. The other end of the second spring 8 is fixed with the wire pressing device 40. The wire pressing device 40 is a rectangular block. The lower side of the wire pressing device 40 is lower than the lower side of the main body of the cutter head in the natural state of the second spring 8.

### Embodiment 7

A device for shaping an angle includes a substrate 1, a support platform 2 provided on the substrate 1, and an angled cutter head provided on the support platform 2. The angled cutter head is fixed movably up and down on the upper side of the support platform 2 via a spring connection device. The support platform 2 and three angled cutter heads with which the support platform cooperates are provided, and fixed in a Chinese character "pin" shape on the substrate 1. The rear side of the support platform 2 is provided with a limit device 5. There is a traveling length between the limit device 5 and the support platform 1. The traveling length is 1mm, 2mm, and 3mm, respectively. The angled cutter head is provided thereon with three different angled cutter heads to facilitate bending the guide wire into a required angle. Correspondingly, the side of the support platform 2 is provided with a guide wire entry platform 4. A plurality of guide wire entry holes 37 are provided on the guide wire entry platform 4. If the guide wire enters from different guide wire entry hole 37, the guide wire corresponds to different angles of the cutter head.

The specific structure can be further described as follows: the substrate 1 is provided thereon with the support platform 2. The front side of the support platform 2 is provided with the wire guide entry platform 4. The left and right sides of the support platform 2 are provided with a fixing post 6. The fixing post is provided with a first spring. An external housing 3 of the angled cutter head is installed on the fixing post. The central part of the external housing 3 of the angled cutter head is provided with a pressing plate 7. The lower side of the pressing plate 7 is provided with two second springs 8 symmetrically along a central axis thereof. The two second springs 8 are connected to the two ends of the angled cutter head 31. The angled cutter head 31 includes a first stepped platform 10 and a second stepped platform 11. The vertical side of the second stepped platform 11 adjacent to the support platform 2 is provided as a certain angle of a slope surface . During the sinking of the angled cutter head 31, the bottom surface of the first stepped platform 10 fits the upper side of the support platform 2 to fix the guide wire. The upper cutter head part on the second stepped platform 11 is pressed down to complete the task of bending the guide wire. A limit device 5 is provided at the rear side of the support platform 2. the positions of the limit device 5 and the support platform 2 can be adjusted according to the actual required length of the bent part of the guide wire.

The technical solution provided by the present invention has the following advantages: 1. The present invention can accurately, stably and quickly bend the guide wire. 2. The bending needs of different angles of the wire guides can be met, that is, the wire guide head can be bent into different angles. The length of a bending part is different. 3. The device can be used a plurality of times for the same patient in the same procedure. The device can be discarded after use, with low costs of use and high safety performance. 4. A set impact travel is precise. A sinking space of the angled cutter head is strictly limited. A moving space of the guide wire is strictly limited. A reaching space of the guide wire is limited, so that a bending guide wire with high precision can be prepared.

## Claims

1. A device for shaping an angle, comprising a support platform and an angled cutter head, wherein the angled cutter head is fixedly connected to the support platform via a connecting device, the angled cutter head can move up and down under constraint of the connecting device to bend the guide wire, the angled cutter head is a three-dimensional structure with a wire pressing surface, the three-dimensional structure is a main body of the cutter head, the pressing surface is an inclined surface, and the connecting device is a spring connecting device.

2. The device for shaping the angle according to claim 1, wherein the wire pressing surface consists of a plurality of different inclined surfaces which act as small cutter heads.

3. The device for shaping the angle according to claim 1, wherein the spring connecting device is structured as follows: a first spring is provided between the angled cutter head and the support platform, the first spring is arranged on a guide post, one end of the guide post is fixed to the support platform, and the other end of the guide post passes through the angled cutter head and is a free end.

4. The device for shaping the angle according to claim 2, wherein a wire guide entry platform is provided on the support platform, a wire guide entry hole is provided on the wire guide entry platform so that the wire guide is guided under the angled cutter head, a plurality of wire guide entry holes are provided on the wire guide entry platform, the number of wire guide entry holes matches the number of small cutter heads of the angled cutter head, and the lowest point of a wire exit of an end of the wire guide entry hole is tangential to a side of the support platform.

5. The device for shaping the angle according to claim 1, wherein the device for shaping the angle is provided with a wire pressing device, the angled cutter head is connected to the wire pressing device via a spring device, a main body of the angled cutter head is provided with an inner cavity, a second spring is provided in the inner cavity, one end of the second spring is fixed with the main body of the cutter head, and the other end of the second spring is fixed with the wire pressing device, a lower side of the wire pressing device is lower than a lower side of the main body of the cutter head in a natural state of the second spring, the inner cavity is arranged in a middle of the angled cutter head, a pair of the second springs are provided and arranged symmetrically along a midline of the inner cavity, and the wire pressing device is a three-dimensional structure of which a bottom surface is a plane.

6. The device for shaping the angle according to claim 4, wherein the device for shaping the angle is provided thereon with a limit device to limit a bending length of the guide wire, the limit device is provided on a rear side of the angled cutter head and an opposite side of the guide wire entry hole, a distance between a front side of the limit device and a rear side of the support platform is called as a traveling length, and the traveling length determines a bending length of the guide wire.

7. The device for shaping the angle according to claim 6, wherein the device for shaping the angle is provided on a substrate, the substrate is provided with mounting structures of a plurality of monomers of the device for shaping the angle, each of the devices for shaping the angles and the limit device form one monomer of the device for shaping the angle, and each of the devices for shaping the angles has a different traveling length.

8. A method for shaping the device for shaping the angle according to claim 6, comprising the following steps: setting a guide wire entry hole at one side of a support platform; setting a plurality of guide wire entry holes; cooperating, by a size of an aperture of the guide wire entry hole, with a thickness of a material to be bent according to actual needs, and the lowest point of a wire exit at an end of the guide wire entry hole being tangential with an upper side of the support platform to ensure that an upper side of the support platform is capable of playing a support role after the guide wire enters the guide wire entry hole; connecting, by the support platform, the angled cutter head together via a spring connecting device, and moving up and down the angled cutter head under constraint of the spring connecting device to bend the guide wire.

9. The method for shaping the device for shaping the angle according to claim 8, wherein a limit device is capable of moving back and forth and withdrawn when the angled cutter head is pressed down for shaping to ensure that an end of the guide wire is not blocked when the guide wire is bent.

10. A device for shaping a guide wire, comprising a support platform and an angled cutter head, wherein the angled cutter head is fixedly connected to the support platform via a connection device, the angular cutter head moves up and down under restraint of the connecting device to bend a guide wire, the angled cutter head is a three-dimensional structure with a wire pressing surface, the three-dimensional structure is a main body of the cutter head, the wire pressing surface is an inclined surface, the connecting device is a spring connecting device, the support platform is provided with a guide wire entry platform so that the guide wire is guided to be below the angled cutter head, the device for shaping the angle is provided thereon with a wire pressing device, the angled cutter head is connected to the wire pressing device via a spring device, a main body of the angled cutter head is provided with an inner cavity, a second spring is provided in the inner cavity, one end of the second spring is fixed with the main body of the cutter head, and the other end of the second spring is fixed with the wire pressing device, a lower side of the wire pressing device is lower than a lower side of the cutter head body in a natural state of the second spring, the inner cavity is arranged in a middle of the angled cutter head, a pair of the second springs are provided and arranged symmetrically along a midline of the inner cavity, and the wire pressing device is a three-dimensional structure of which a bottom surface is a plane.
